Europäisches Patentamt

(19) European Patent Office

Office européen des brevets

(11) Publication number: **0 112 307**
**B1**

(12) **EUROPEAN PATENT SPECIFICATION**

(45) Date of publication of patent specification: **26.07.89**

(21) Application number: **83830264.4**

(22) Date of filing: **12.12.83**

(51) Int. Cl.⁴: **A 61 M 16/00**

(54) **A tracheal intubation cannula with external valve.**

(30) Priority: **22.12.82 IT 363182**

(43) Date of publication of application:
**27.06.84 Bulletin 84/26**

(45) Publication of the grant of the patent:
**26.07.89 Bulletin 89/30**

(84) Designated Contracting States:
**AT BE CH DE FR GB LI NL SE**

(56) References cited:
**EP-A-0 078 685**
**CH-A- 408 292**
**DE-A-2 505 123**
**DE-U-1 852 111**
**US-A-2 039 142**
**US-A-3 137 299**
**US-A-3 827 440**
**US-A-3 924 637**
**US-A-4 223 411**
**US-A-4 325 366**

(73) Proprietor: **Cavalli, Walter**
**Via dell'Artigiano, 11**
**I-40055 Villanova di Castenaso (Bologna) (IT)**
(73) Proprietor: **Rangoni, Marco**
**Via delle Frangole, 45**
**Bologna (IT)**
(73) Proprietor: **Cavalli, Dante**
**Via dell'Artigiano, 9**
**I-40055 Villanova di Castenaso (Bologna) (IT)**
(73) Proprietor: **Rangoni, Roberto**
**Via Venezia, 20**
**S. Lazzaro di Savena (Bologna) (IT)**

(72) Inventor: **Cavalli, Walter**
**Via dell'Artigiano, 11**
**I-40055 Villanova di Castenaso (Bologna) (IT)**
Inventor: **Rangoni, Marco**
**Via delle Frangole, 45**
**Bologna (IT)**
Inventor: **Cavalli, Dante**
**Via dell'Artigiano, 9**
**I-40055 Villanova di Castenaso (Bologna) (IT)**
Inventor: **Rangoni, Roberto**
**Via Venezia, 20**
**S. Lazzaro di Savena (Bologna) (IT)**

(74) Representative: **Pozzoli, Giuliano**
**e/o ENGIMPEX S.A. Industrial Property Services**
**Dept. P.O. Box 12**
**CH-6924 Lugano-Sorengo (CH)**

Courier Press, Leamington Spa, England.

## Description

This invention relates to a tracheal tube fitted with a mechanically operated valve intended specifically for patients who have undergone a tracheotomy, especially where it is impossible to rebuild the upper airways and where consequently the tracheostoma must be maintained. It is known that by means of this procedure the air enters the lungs via the opening made in the trachea and is expelled by the same route, thus bypassing the upper airways and this obviously does not give the patient any possibility of speech. It is also known that these patients, in order to produce any vocal sounds, must manually close off the opening in the trachea, thus directing the flow of air over the vocal cords (if these are still present).

It is known to overcome this disadvantage by means of a valve fixed to the external end of the tube: this valve is able to interrupt the outflow of air depending on the force with which this air is expelled. A tracheal intubation cannula with such a valve is known from US-A-4 325 366, which discloses a tracheal intubation cannula comprising a valve with a cylindrical body having a front plate which faces forwards when positioned on the patient, the front plate including at least one opening to allow air to flow into and out of the cannula, the valve being mounted on the top end of the cannula designed to be positioned external to the patient and designed to cut off the air exhaled from the lungs only when the exhalation is vigorously emitted i.e. more so than in normal breathing and equal to that necessary for speech.

The present invention also reduces other problems found with traditional tracheotomy tubes without valves, such as the very high possibility of bacterial contamination. The device which is the subject of this invention is essentially characterised in that the cannula is shaped as a truncated one to facilitate introduction into an incision in the patient's trachea and in that openings to allow air to flow into and out of the cannula are located also in the cylindrical side walls.

The top extremity may have a small collar suitably shaped to fit over the opening in the neck without causing irritation due to the unavoidable movement that occurs between the parts in contact. At the same time, this collar may provide the recess for the valve, which may be fitted to it by means of a spring-clip. The valve cylindrical body may end with two projections at the ends of which may be fixed the front plate with an area less than that of the cylinder end. Inside the cylinder body may be fixed a circular ring to act as the valve seat and kept in place by means of a projecting ridge.

The said valve may be kept open by a spiral spring placed between the valve opening and the top plate; the top plate may have a central stud with a hole through it, which together with a second similar stud placed at the centre of the valve flap keeps the two ends of the spring in place.

The top plate can have holes drilled in it to facilitate the passage of air, or else in order to enable a covering ornamental medallion (which would disguise the endotracheal tube opening) to be fixed to it by means of screws. Two supporting rings may be provided on the medallion collar to enable a chain (which would go round the patient's neck) to be fitted and this chain completes the ornamental effect but much more importantly prevents the tube from accidentally falling down to the floor.

These and other characteristics will now be described in greater detail using a simple example of the device which is however intended to be purely illustrative and does not limit in any way the scope of this invention.

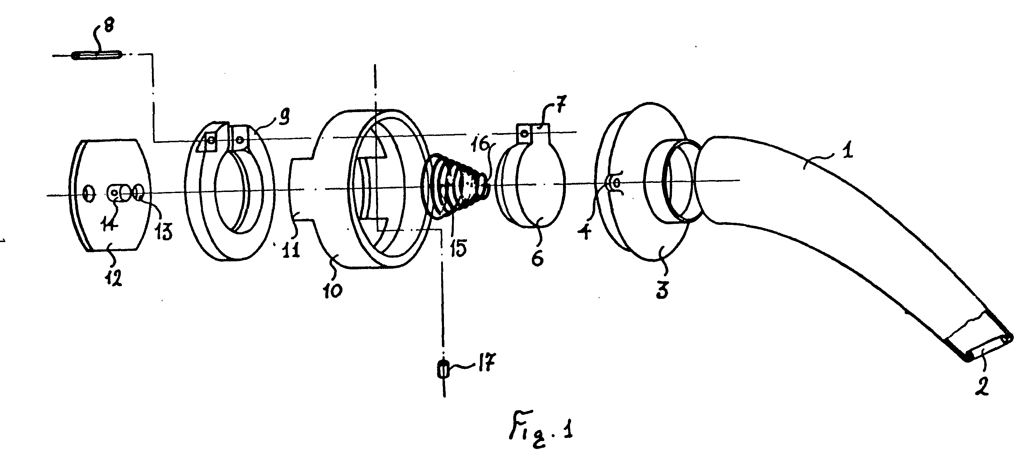

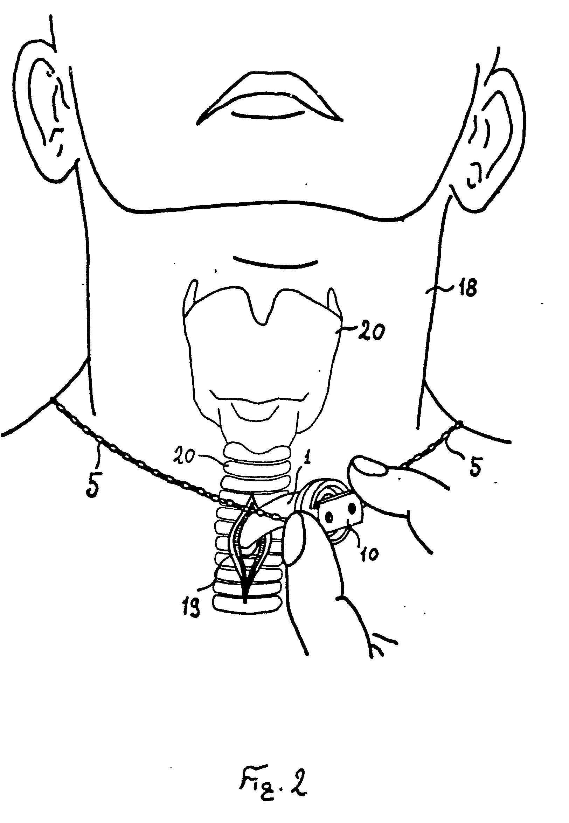

With reference to the accompanying drawings, in Fig. 1 the device is shown in exploded view while in Fig. 2 the device is shown as being positioned on the patient; in the drawings, reference numeral 1 shows the cannula, 2 is the rounded inner edge, 3 is the collar, shaped so as to achieve a non-irritant contact, 4 are the rings to which the chain 5 is attached. Reference numeral 6 is the disc-shaped valve flap, 7 is the projection for the pin 8 which joins the valve flap to its seat 9, 10 is the outer casing of the valve while 11 are the projections to which the front plate 12 is fixed, 13 are the holes (intended for a number of uses) drilled in the front plate, 14 is the central perforated stud which holds one end 15 of the spring while the other end 16 is kept in place by a similar stud located at the centre of the valve flap but not shown in the drawing, 17 is the pawl of the release mechanism the parts of which are not shown in the drawing but which are located inside the collar. In Fig. 2, 18 is the neck of the patient on whom a tracheostoma 19 has been carried out, while 20 shows the trachea.

The device works as follows: during respiration the patient breathes in air through the valve which is kept open by the spring and the following expiration takes place always through the same opening so that air from the lungs does not reach the upper airways.

During speech the patient, by emitting the air with greater force, overcomes the pressure exerted by the spring and thus closes the valve. Hence the air, since it cannot escape through the tracheostoma opening, is directed upwards thus reaching the vocal cords.

## Claims

1. A tracheal intubation cannula comprising a valve with a cylindrical body (10) having a front plate (12) which faces forwards when positioned on the patient, the front plate including at least one opening to allow air to flow into and out of the cannula, the valve being mounted on the top end of the cannula designed to be positioned external to the patient and designed to cut off the air exhaled from the lungs only when the exhalation is vigorously emitted, i.e. more so than in normal breathing and equal to that necessary for speech, characterized by the fact that the cannula is shaped as a truncated cone to facilitate introduction into an incision in the patient's trachea and in that openings to allow air to flow into and out of the cannula are located also in the cylindrical side walls.

2. A tracheal intubation cannula with external valve as set forth in Claim 1, characterized by the fact that the valve has a circular flap (6) which is seated on a circular crown (9) which is interrupted to permit insertion of a projection (7) on the valve flap, the two parts being connected together by means of a pin (8) inserted into the projection.

3. A tracheal intubation cannula with external valve as set forth in Claim 2, characterized by the fact that the valve flap is positioned so as to define a front face and that at the centre of each of the front plate (12) of the valve body and of the front face of the valve flap (6) is found a projecting stud (14), each with a hole through it, which keeps in position a spiral spring (15) which holds the valve flap open (6), except when the valve operates to interrupt the outflow of air.

4. A tracheal intubation cannula with external valve as set forth in any one of Claims 1 to 3, characterized by the fact that the cannula (1) is curved and is of a tapered section diminishing towards the internal aperture, the edges of which are suitably rounded to facilitate easy introduction of the device.

5. A tracheal intubation cannula with external valve as set forth in any one of Claims 1 to 4, characterized by the fact that the cannula has at its upper end a collar (3) which is provided with rings (4) for the attachment of a supporting chain (5) which is also ornamental.

6. A tracheal intubation cannula with external valve as set forth in Claim 5, characterized by the fact that the front plate (12) has holes (13) in it to improve air flow, but also to allow the attachment of a medallion which hides the tracheotomy tube from view, and which appears to be held in place by the chain round the patient's neck.

## Patentansprüche

1. Eine Trachealintubationskanüle, die ein Trommelventil (10) mit einer Stirnplatte (12) enthält, die, wenn sie auf dem Patient gestellt ist, zu vorne hinschaut und mindestens ein Loch enthält, um die Luft in die Kanüle ein- und ausfließen zu lassen, während das Ventil am oberen Ende der Kanüle montiert ist und so konstruiert ist, um außen dem Patient gestellt zu werden und um die von den Lungen ausgeatmte Luft zu unterbrechen, nur wenn die Ausatmung kräftig (nämlich mehr als diese in normaler Atmumg erfordet und gleich zu diese zu sprechen nötig) ist, die von der Tatsache charakterisiert ist, daß die Kanüle abgestumpft ist, um die Einführung in eine Einschnitt in der trachea des Patients zu erleichtern und daß die Löcher, die den Ein- und Auslaß der Luft von der Kanüle erlauben, auch in den zylindrischen Seitenwänden gestellt sind.

2. Eine Trachealintubationskanüle mit Außenventil gemäß dem Anspruch 1, die von der Tatsache charakterisiert ist, daß das Ventil eine scheibeförmige Klappe (6) hat, die in einer kreisförmigen Sitz (9) liegt, der untergebrochen ist, um die Einfügung einer Vorsprung (7) in der Ventilklappe zu ermöglichen, die beiden Teile sind durch einen Bolzen (8), der in der Vorsprung eingefügt ist, verbunden.

3. Eine Trachealintubationskanüle mit Außenventil gemäß dem Anspruch 2, die von der Tatsache charakterisiert ist, daß die Ventilklappe so gestellt ist, daß eine Vorderseite festgesetzt wird und daß ein Hohlbolzen (14) in der Mitte von jeder Stirnplatte (12) des Ventilkörpers und in der Vorderseite der Ventilklappe (6) liegt und eine Spiralfeder (15) in Stellung hält, die die Ventilklappe (6) geöffnet hält, wenn das Ventil die Luftauslaß nicht unterbricht.

4. Eine Trachealintubationskanüle mit Außenventil gemäß dem Anspruch entweder 1 oder 2 oder 3, die von der Tatsache charakterisiert ist, daß die Kanüle (1) gebogen ist und daß sie einen verjüngten Querschnitt hat, der nach der Innenöffnung sinkt, deren Ränder angemessen abgerundet sind, um die Einführung des Geräts zu erleichtern.

5. Eine trachealintubationskanüle mit Außenventil gemäß dem Anspruch entweder 1 oder 2 oder 3 oder 4, die von der Tatsache charakterisiert ist, daß sie am ihrem Oberende einen Kragen (3) hat, der mit Ringe für die Befestigung einer schmückenden Stützkette (5) ausgerüstet ist.

6. Eine Trachealintubationskanüle mit Außenventil gemäß dem Anspruch 5, die von der Tatsache charakterisiert ist, daß die Stirnplatte (12) gelocht (13) ist, um den Durchfluß der Luft zu erleichtern und um die Befestigung eines Medaillons zu ermoglichen, das die Öffnung des Trachealrohrs versteckt und das von der Kette um dem Hals des Patients in Stellung gehalten wird.

## Revendications

1. Une canule d'intubation trachéale comprenant une valve à corps cylindrique (10) ayant une plaque frontale (12) qui est tournée en avant quand elle est en position sur le patient, la plaque frontale comprenant au moins une ouverture pour permettre à l'air de s'écouler dans la canule et d'en sortir, la valve étant montée à l'extremité supérieure de la canule projetée pour être positionnée à l'exterieur du patient et projetée pour couper l'air expirée par les poumons seulement quand l'expiration est emise avec vigueur, c'est-à-dire plus vigoureusement que dans la respiration normale et avec la force necessaire pour parler, caractérisée par le fait que la canule est coupée à cône pour faciliter son introduction dans une incision dans la trachée du patient et par le fait que des ouvertures pour permettre à l'air de s'écouler dans la canule et d'en sortir sont placées aussi dans les parois cylindriques latérales.

2. Une canule d'intubation trachéale avec valve externe d'après la revendication 1, caractérisée par le fait que la valve a un volet circulaire (6) qui est logé dans une couronne circulaire (9) qui s'interrompt pour permettre l'insertion d'une projection (7) sur le volet de la valve, les deux parts étant jointes par un pivot (8) integré dans la projection.

3. Une canule d'intubation trachéale avec valve externe d'après la revendication 2, caractérisée par le fait que le volet de la valve est positionné de telle sorte qu'il définit une face frontale et par le fait qu'au centre de chaque plaque frontale (12) du corps de la valve et de la face frontale du volet de la valve (6) est placé un pivot creux (14), qui retien en place un ressort à boudin (15) qui tient ouvert le volet de la valve (6), sauf que la valve agit pour couper l'écoulement de l'air vers l'exterieur.

4. Une canule d'intubation trachéale avec valve externe d'après n'importe quelle des revendications 1, 2 ou 3, caractérisée par le fait que la canule (1) est courbée et a une section qui présente une contracture vers l'ouverture interne, dont les bords sont opportunément arrondis pour faciliter l'introduction de l'appareil.

5. Une canule d'intubation trachéale avec valve externe d'après n'importe quelle des revendications 1, 2, 3 ou 4, caractérisée par le fait que la canule a à son extremité supérieure un collet (3) avec des anneaux (4) pour la mise en place d'une chaîne de support (5) qui est aussi ornementale.

6. Une canule d'intubation trachéale avec valve externe d'après la revendication 5, caractérisée par le fait que la plaque frontale (12) a des trous (13) pour améliorer l'écoulement de l'air, mais aussi pour permettre la mise en place d'une médaille qui cache le tube de trachéotomie et qui est retenue en place par la chaîne autour le cou du patient.

Fig. 1

EP 0 112 307 B1

Fig. 2